# FASCICULE DE BREVET EUROPEEN

(11) **EP 2 970 845 B1**
(45) Date de publication et mention de la délivrance du brevet: **25.09.2019**
(21) Numéro de dépôt: 14713553.7
(22) Date de dépôt: 12.03.2014
(51) Int. Cl.: C12M 1/00

(54) **BIOREACTEUR**
BIOREAKTOR
BIOREACTOR

(30) Priorité: 13.03.2013 FR 1352236
(43) Date de publication de la demande: 20.01.2016
(73) Titulaire: Roquette Frères, 62136 Lestrem (FR)
(72) Inventeur: SEGARD, David, F-62136 Lestrem (FR)
(74) Mandataire: Cabinet Plasseraud
(86) Numéro de dépôt international: PCT/FR2014/050555
(87) Numéro de publication internationale: WO 2014/140476

(56) Documents cités:
- CN-U- 201 476 623
- DE-A1- 2 753 388
- US-A- 3 978 918

## Description

L'invention concerne tout type de bioréacteurs et trouvera une application particulière pour la mise en oeuvre de fermentations complexes, sensibles aux contaminations, voire même pour la mise en oeuvre de réactions biochimiques ou biologiques.

Le domaine de l'invention est celui des bioréacteurs, plus particulièrement celui des fermenteurs permettant la mise en oeuvre de fermentations discontinues, dites de type *batch*, semi-continues, dites de type *fed-batch* ou continues.

L'invention s'intéresse plus particulièrement aux réacteurs, aux échelles pilotes ou industrielles, permettant de grands volumes de production dont les cuves peuvent atteindre des capacités comprises entre 20 m³ et plusieurs centaines de m³.

Les bioréacteurs sont utilisés pour produire de la biomasse cellulaire (levures, bactéries, champignons microscopiques, microalgues, cellules animales et végétales), et/ou pour assurer la production de métabolites d'intérêt (protéines, vitamines, antibiotiques, polyols...).

L'état de la technique connaît ainsi des réacteurs, aux échelles pilotes ou industrielles, trouvant une application particulière pour cette utilisation.
De tels réacteurs comprennent classiquement un corps de cuve, de grand volume, bien souvent cylindrique, à axe vertical, à l'intérieur duquel se déroule la réaction biologique (fermentation) ou biochimique (enzymatique), souvent sous forte agitation.

Des moyens d'agitation sont prévus, intérieurs au corps de cuve et comprennent classiquement un rotor, généralement d'axe coaxial à celui du corps de cuve, supportant des pales destinées à brasser fortement la biomasse. Ce rotor est entrainé en rotation par rapport au corps de cuve par un motoréducteur. Les moyens d'agitation comprennent également des contre-pales, saillantes vers le centre de la cuve, solidarisées autour du rotor en des positions fixes par rapport au corps de cuve.

Afin de pouvoir contrôler la température de la réaction, il est par ailleurs connu de refroidir ou de chauffer le réacteur par l'extérieur et/ou par l'intérieur, au moyen d'un ou plusieurs échangeur(s), externe(s) ou interne(s) au corps de cuve. Dans chaque échangeur circule un fluide, caloporteur et/ou frigoporteur, selon les besoins.

Bien souvent, lors de la réaction de fermentation, exothermique, un ou plusieurs groupes froids permettent la circulation d'un fluide frigoporteur dans les échangeurs, et évitent ainsi que la température de réaction ne soit trop élevée.
L'échangeur externe à la cuve peut être constitué par une double enveloppe du corps de cuve à l'intérieur de laquelle circule le fluide caloporteur/frigoporteur. Un échangeur, externe, présente pour avantage de laisser libre le volume intérieur du corps de cuve. En revanche, la performance de l'échange thermique est inférieure à celle d'un échangeur interne.

C'est la raison pour laquelle un échangeur interne à la cuve est souvent nécessaire, lorsque le contrôle en température de la réaction nécessite un fort échange thermique. Les échangeurs internes comprennent une ou plusieurs conduites s'étendant dans le volume interne du corps de cuve. La paroi externe des conduites de l'échangeur interne est en contact direct avec la biomasse.

L'état de la technique connait ainsi un échangeur interne qui se présente sous la forme d'une conduite s'étendant sous la forme d'une hélicoïde, encore appelé « serpentin », l'axe de l'hélicoïde étant généralement d'axe coaxial au corps de cuve.

Lors de la réaction à l'intérieur de la cuve, cette conduite est susceptible de se déformer fortement sous l'action des contraintes de pression dues à la forte agitation interne, ainsi que des contraintes dues à la rétractation et/ou à la dilatation de la conduite, engendrées par les variations de températures.

Afin de limiter cette déformation, il est connu de supporter cette conduite, non seulement au niveau du piquage d'entrée, et du piquage de sortie, mais également en différents points de réception le long de l'hélicoïde.

Selon l'état de la technique connu, le support du serpentin est classiquement réalisé au moyen d'étriers (en anglais « *U bolt »)* venant à cheval sur la conduite, au niveau des différents points de réception le long de l'hélicoïde, et fixés à la paroi interne, directement, ou indirectement par l'intermédiaire de supports.

Le document CN201476623U divulgue et illustre une variante de ce mode de fixation de la conduite hélicoïdale par étriers.

Un tel réacteur, comprenant un serpentin interne supporté par étriers, est couramment utilisé pour des fermentations par batch. La demanderesse utilise ainsi de tels réacteurs pour la production de métabolites d'intérêt.

La demanderesse a utilisé récemment de tels bioréacteurs pour une nouvelle application, à savoir la production de biomasses de cellules, et plus particulièrement la culture de microalgues, du genre *Chlorella.*

Plus particulièrement, l'objectif était de réaliser une production de la biomasse de microalgues en conditions hétérotrophiques, i.e. à l'obscurité, en présence d'une source carbonée assimilable par ladite microalgue.

Lors de cet essai, ces organismes unicellulaires se sont révélés très sensibles aux contaminations qui, lorsqu'elles sont apparues, ont pris rapidement le dessus sur les organismes cultivés. En effet, la vitesse de croissance des contaminants, notamment bactériens, est bien plus importante que celles des microalgues.

Pour l'instant il n'existe pas de solutions économiquement viables pour séparer la biomasse de microalgues de ses contaminants. Aussi, en pratique, lorsqu'une contamination apparait, le contenu du bioréacteur est entièrement vidé et mis au rebut, le réacteur devant ensuite être nettoyé et stérilisé avant la mise en oeuvre d'une nouvelle réaction de fermentation.

Après divers essais, et selon les constatations de l'inventeur, il est apparu à la demanderesse que la configuration des bioréacteurs à échangeur interne de l'état de la technique est trop favorable à l'apparition et au développement de contaminations, et donc inadaptés à la culture de longue durée d'organismes unicellulaires en particulier de type microalgues.

Le but de la présente invention est de pallier les inconvénients précités en proposant un bioréacteur, apte à permettre le contrôle de la température de la réaction interne à la cuve, de performance thermique satisfaisante, permettant la mise en oeuvre de fermentations complexes, sensibles aux contaminations.
Plus particulièrement, le but de la présente invention est de proposer un tel réacteur qui évite, ou à tout le moins limite les interruptions de production.

D'autre buts et avantages de l'invention apparaîtront plus clairement au cours de la description suivante qui n'est donnée qu'à titre indicatif et qui n'a pas pour but de la limiter.

Aussi l'invention concerne un bioréacteur comprenant :
- un corps de cuve, dont les parois internes définissent entre elles un volume intérieur pour la réception de la biomasse,
- une conduite de refroidissement et/ou de chauffage dont la paroi extérieure est destinée à être en contact direct avec la biomasse, disposée dans ledit volume intérieur, et s'étendant au moins sur une partie de longueur sous la forme d'une hélicoïde,
- des moyens de fixation assurant le support de ladite conduite de refroidissement et/ou de chauffage et sa fixation au corps de cuve, en plusieurs positions de réception le long de l'hélicoïde.

Selon l'invention, lesdits moyens de fixation comprennent :
- des raccords, de préférence tubulaires ou semi tubulaires, enveloppant ladite conduite par l'extérieur, localement, au niveau desdites différentes positions de réception le long de l'hélicoïde,
- un ou plusieurs supports (lames, profilés...), solidarisés à la paroi interne du corps de cuve, saillants vers le centre du corps de cuve , ledit ou lesdits supports présentant plusieurs alésages de forme complémentaire aux dimensions externes des raccords, prévus au niveau desdites différentes positions de réception le long de l'hélicoïde, lesdits alésages recevant lesdits raccords,
et dans lequel ladite conduite de refroidissement et/ou de chauffage est solidarisée au niveau de chacune desdites positions de réception par l'intermédiaire :
- d'une première soudure reliant le raccord correspondant et le support (lame, profilé...) correspondant, ladite première soudure s'étendant le long des bords de l'alésage correspondant, des deux côtés dudit support (lame, profilé...) correspondant, et,
- d'une seconde soudure reliant le raccord correspondant et la conduite de refroidissement et/ou de chauffage, ladite seconde soudure s'étendant le long des bords dudit raccord.

De préférence, ladite première soudure et ladite deuxième soudure sont des soudures par apport de métal.

Il est bien entendu que le terme « soudure » utilisé peut comprendre un cordon de soudure ou plusieurs cordons, comme il est connu de l'homme du métier en toute généralité.

Selon des caractéristiques optionnelles, prises seules ou en combinaison :
- le cordon de métal ou chacun des cordons de métal de ladite première soudure et/ou de ladite deuxième soudure forme un congé, en creux, lesdites soudures étant de préférence polies ;
- chacun des alésages est débouchant au niveau d'un bord dudit support (lame, profilé...) ou de l'un desdits supports (lames, profilés...) ;
- les alésages sont débouchant vers le centre du corps de cuve ;
- ladite première soudure est constituée par une ligne de soudure continue, de trajectoire fermée, qui court, au niveau des bords de l'alésage, des deux côtés dudit support (lame, profilé...) correspondant, ainsi que sur le chant dudit support (lame, profilé...) correspondant, en partie supérieure et en partie inférieure de ladite conduite de refroidissement et/ou de chauffage ;
- les raccords sont de forme semi-tubulaire et dans lequel ladite seconde soudure est constituée par une ligne de soudure continue, de trajectoire fermée, qui court, au niveau des bords du raccord, le long des bords longitudinaux du raccord, ainsi que le long des bords arqués dudit raccord ;
- lorsque le bioréacteur présente des moyens d'agitation, ils comprennent, d'une part, un rotor, intérieur au corps de cuve, mobile en rotation par rapport au corps de cuve, le rotor présente des pales, et d'autre part, des contre-pales, saillantes vers le centre de la cuve, solidarisées en des positions fixes par rapport au corps de cuve ;
- les contre-pales desdits moyens d'agitation peuvent comprendre lesdits supports (lames, profilés...) ou ledit support (lame, profilé...) desdits moyens de fixation (comme il sera exemplifié ci-après) ;
- les alésages sont dits premiers alésages, ledit ou lesdits supports présentant, outre lesdits premiers alésages au niveau desdites différentes positions de réception de l'hélicoïde, des seconds alésages, de dimensions surabondantes par rapport au diamètre de ladite conduite, lesdits seconds alésages étant traversés par ladite conduite de telle façon à laisser un jeu autorisant la libre déformation de ladite conduite ;
- le réacteur présente plusieurs dits supports (lames, profilés...), répartis angulairement sur la circonférence interne du corps de cuve, orientés chacun suivant leur axe longitudinal selon la hauteur du corps de cuve, s'étendant chacun en correspondance sur la hauteur de l'hélicoïde ;
- ledit support (lame, profilé...) ou chacun des supports (lames, profilés...) présente un bord extérieur en vis-à-vis de la paroi interne, latérale, de la cuve et dans lequel lesdits moyens de fixation comprennent des contreventements assurant la solidarisation dudit support (lame, profilé...) ou de chacun desdits supports (lames, profilés...), à la paroi interne, latérale, du corps de cuve, en créant un inter-espace entre le bord extérieur dudit support (lame, profilé...) correspondant et la paroi interne du corps de cuve ;
- chaque contreventement est soudé, respectivement au dudit support (lame, profilé...) correspondant et à la paroi interne du corps de cuve et dans lequel lesdites soudures, par apport de métal, présentent un cordon de métal formant un congé en creux, lesdites soudures étant polies.
- le bioréacteur peut être pourvu d'un dispositif de refroidissement/chauffage du corps de cuve par l'extérieur, par circulation d'un fluide frigoporteur/caloporteur.

L'invention sera mieux comprise à la lecture de la description suivante accompagnée des dessins parmi lesquels :
- La figure 1 est une vue en transparence d'un bioréacteur conforme à l'invention selon un mode de réalisation pour lequel les supports prennent la forme de lames,
- La figure la est une vue de dessous du bioréacteur selon la figure 1,
- La figure 2 est une vue de détail, de côté, selon une coupe verticale, illustrant lesdits moyens de fixation selon un mode de réalisation de l'invention,
- La figure 3 est une vue de face desdits moyens de fixation tels qu'illustrés à la figure 2,
- La figure 4 est une vue de dessus desdits moyens de fixation tels qu'illustrés à la figure 2,
- La figure 5 est une vue de détail illustrant, au niveau de l'un des points de réception de l'hélicoïde, ladite première et deuxième soudure,
- La figure 6 est une vue de face du point de réception de l'hélicoïde de la figure 5,
- La figure 7 est une vue selon la coupe VI-VI de la figure 6, et la figure 7a est une vue de détail de la figure 7,
- La figure 8 est une vue selon la coupe VII-VII de la figure 7,
- La figure 9 est une vue de détail d'un raccord semi tubulaire,
- La figure 10 est une vue de détail d'un raccord tubulaire, enveloppant la conduite,
- La figure 11 est une vue en perspective d'un support prenant la forme d'un profilé semi-tubulaire,
- La figure 12 est une vue de dessus de la figure 11,
- La figure 13 est une vue selon la coupe XII-XII telle qu'illustrée à la figure 12,
- La figure 14 est une vue de face de la figure 11.

L'invention est née de la constatation par l'inventeur que les bioréacteurs à échangeur(s) interne(s) de l'état de la technique créent, au sein du corps de cuve du réacteur, de nombreuses zones interstitielles et ou des zones non vidangeables, favorables aux dépôts de matière notamment organiques et sur lesquels apparaissent et se développent les contaminations.
Selon les constatations de l'inventeur, ces zones interstitielles se trouvent, en grande partie, au niveau de l'échangeur et/ou de ses zones de réception.

En particulier dans le cas d'échangeur interne à serpentin, ces zones interstitielles et/ou des zones non nettoyables sont situées sur la structure à étriers supportant la conduite hélicoïdale, en particulier au niveau de chaque interstice formé entre l'un des étriers et la conduite et aussi au niveau des supports.

Afin de remédier à ce problème, la présente demanderesse aurait pu choisir de supprimer simplement cet échangeur interne, au bénéfice d'un échangeur externe, mais cette solution n'a pas été retenue en raison du moins bon coefficient de transfert d'un échangeur externe et également en raison de la complexité à maintenir ce type d'installation dans des conditions hygiéniques et surtout aseptiques satisfaisantes.

La présente demanderesse a au contraire décidé de concevoir un bioréacteur dont l'échangeur interne et son support, évitent, ou à tout le moins limitent fortement ou empêchent les risques de développement de contaminants au sein du réacteur tout en facilitant sa nettoyabilité.

Aussi l'invention est relative à un bioréacteur 1 comprenant :
- un corps 2 de cuve, dont les parois internes définissent entre elles un volume intérieur pour la réception de la biomasse,
- une conduite 3,3',3" de refroidissement et/ou de chauffage dont la paroi extérieure est destinée à être en contact direct avec la biomasse, disposée dans ledit volume intérieur, et s'étendant au moins sur une partie de longueur sous la forme d'une hélicoïde,
- des moyens de fixation 4 assurant le support de ladite conduite 3,3',3" de refroidissement et/ou de chauffage et sa fixation au corps 2 de cuve, en plusieurs positions de réception le long de l'hélicoïde.

La conduite 3,3', 3" de refroidissement et/ou de chauffage est donc supportée, non seulement au niveau du piquage d'entrée et du piquage de sortie, mais également en lesdites plusieurs positions de réception.

Un fluide frigoporteur ou caloporteur, notamment de l'eau, est destiné à circuler dans ladite conduite, le fluide frigoporteur pouvant être obtenu au moyen d'un groupe froid ou au moyen d'une chaudière ou de tout autre moyen permettant le retrait ou l'apport de calories.

Le corps de cuve est, de préférence, à paroi latérale cylindrique. Le bioréacteur peut comprendre une conduite de refroidissement et/ou de chauffage, ou encore plusieurs conduites 3,3',3" selon le mode de réalisation non limitatif illustré. La ou les hélicoïdes sont de préférence d'axe coaxial à l'axe du corps de cuve cylindrique.

Ces conduites 3,3',3" peuvent s'étendre respectivement sur trois niveaux distincts de hauteur, de préférence sans recouvrement, les conduites hélicoïdales étant de préférence de mêmes diamètres. Eventuellement, selon un autre mode de réalisation non illustré, les conduites hélicoïdales, de même diamètre, peuvent être imbriquées.

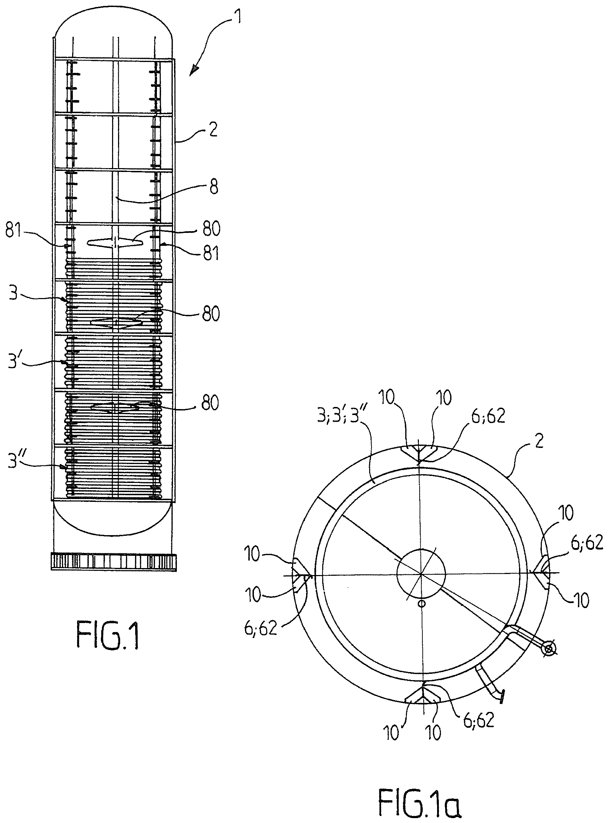

Lesdites conduites 3,3',3" des échangeurs internes peuvent être situées sur la partie basse du corps de cuve seulement, par exemple sur la moitié inférieure selon le mode de réalisation illustré à la figure 1, le bioréacteur étant dépourvu d'échangeur interne en partie supérieure de la cuve.

Selon l'invention, lesdits moyens de fixation comprennent :
- des raccords 5, de préférence tubulaires ou semi tubulaires, enveloppant ladite conduite 3,3',3" par l'extérieur, localement, au niveau desdites différentes positions de réception le long de l'hélicoïde,
- un ou plusieurs supports 6, solidarisés à la paroi interne du corps 2 de cuve, saillants vers le centre du corps de cuve, ledit ou lesdits supports présentant plusieurs alésages 7 de forme complémentaire aux dimensions externes des raccords 5, prévus au niveau desdits différentes positions de réception le long de l'hélicoïde, lesdits alésages 7 recevant lesdits raccords 5.

Ladite conduite 3,3',3" de refroidissement et/ou de chauffage est solidarisée au niveau de chacune desdites positions de réception par l'intermédiaire :
- d'une première soudure S1 reliant le raccord 5 correspondant et ledit support 6 correspondant, ladite première soudure S1 s'étendant le long des bords de l'alésage 7 correspondant, des deux côtés dudit support 6 correspondant, et,
- d'une seconde soudure S2 reliant le raccord 5 correspondant et la conduite 3, 3',3" de refroidissement et/ou de chauffage, ladite seconde soudure S2 s'étendant le long des bords dudit raccord 5.

Ladite première soudure S1 et ladite deuxième soudure S2 sont des soudures par apport de métal. Les cordons de métal des première et deuxième soudures S1, S2 permettent avantageusement de supprimer toute zone interstitielle entre, d'une part, la conduite 3,3',3" et le raccord 5 et, d'autre part, le raccord 5 et le support 6.

Selon l'invention, la conduite est ainsi soudée audit support ou à chacun des supports 6, indirectement, par l'intermédiaire des raccords 5 qui s'étendent chacun en longueur, localement, le long de la conduite 3,3',3", de préférence de part et d'autre du support 6 correspondant. Cette disposition permet de choisir une épaisseur de conduite inférieure à celle qui serait nécessaire si la conduite était soudée directement audit support, sans raccord.

De préférence, le cordon de métal ou chacun des cordons de métal de ladite première soudure S1 et/ou de ladite deuxième soudure S2 forme un congé 11, en creux, lesdites soudures S1, S2 étant de préférence polies.

Lors des opérations de fixation de la conduite, le soudeur réalise, dans un premier temps au moins un cordon de soudure pour la première soudure S1 et au moins un deuxième cordon de soudure pour la deuxième soudure S2. Ces cordons sont ensuite meulés pour former les congés 11 en creux. De tels congés permettent de supprimer les angles vifs qui constituent autant d'amorces à la rupture et permettent d'assurer une meilleure nettoyabilité. Les congés 11 présentent à cet effet un rayon, de préférence supérieur à 5 mm. Les soudures sont ensuite polies afin de supprimer les aspérités.

La liaison mécanique ainsi réalisée entre la conduite 3,3',3" et le ou lesdits supports 6 est avantageusement sans aspérité, et sans zone interstitielle favorisant les dépôts notamment organiques.

Le ou les supports 6 peuvent prendre la forme de lames 62 (voir à titre d'exemple non limitatif les figures 1 à 8), ou encore la forme de profilés 63, tubulaires ou semi-tubulaires.

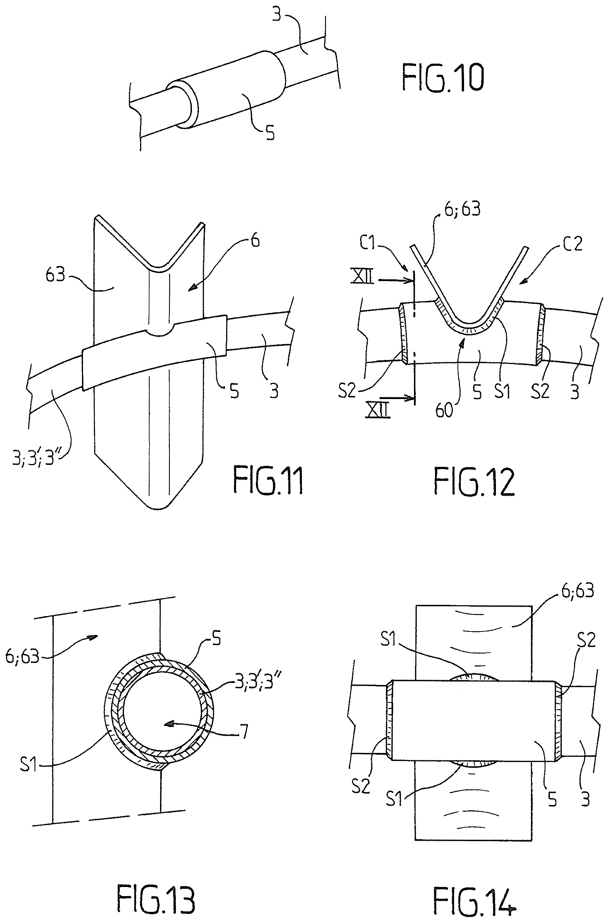

Selon l'exemple non limitatif des figures 11 à 14, le profilé 63 peut être semi tubulaire de section en V, le ou les alésages 7 étant débouchant situés au niveau du sommet du V.

Selon un mode de réalisation (non illustré) chacun des alésages peut être de section fermée, sensiblement circulaire, les raccords étant alors tubulaires. Dans ce mode de réalisation, chaque raccord est soudé audit support (lame, profilé...) grâce à deux cordons de soudures circulaires, distincts, de ladite première soudure S1. Ces deux cordons de soudure sont situés de chaque côté C1, C2 du support (lame, profilé...), joignant la paroi cylindrique du raccord et le support (lame, profilé...), le long de l'alésage circulaire. De plus chaque raccord est soudé à la conduite grâce à deux cordons circulaires de la deuxième soudure joignant les deux bords circulaires du raccord à la conduite de refroidissement et/ou de chauffage.

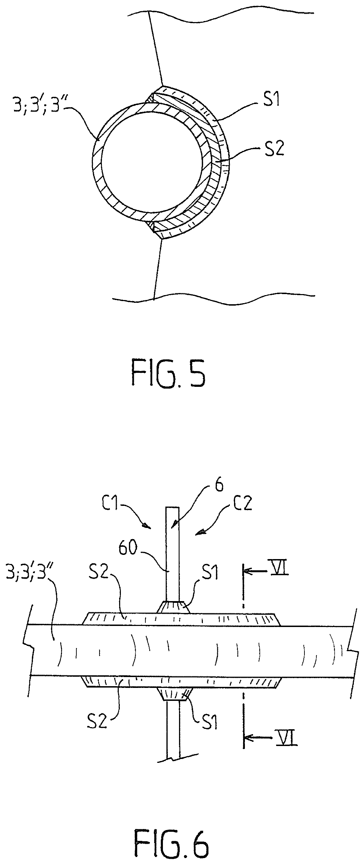

Selon un autre mode de réalisation illustré, facilitant l'assemblage de la conduite sur son support, chacun des alésages 7, de forme semi circulaire, est débouchant au niveau d'un bord du support 6 ou de l'un des supports 6 (lame 62, profilé 63...).
Par exemple et selon les modes de réalisation illustrés, les alésages 7 sont débouchant vers le centre du corps 2 de cuve.
Ladite première soudure S1 peut alors être constituée par une ligne de soudure continue, de trajectoire fermée, qui court (voir figures 5 à 8 dans le cas où les supports sont des lames 62, et figures 12 à 14 dans le cas où les supports sont des profilés 63), au niveau des bords de l'alésage 7, semi circulaire, des deux côtés C1,C2 du support 6 correspondant, ainsi que sur le chant 60 du support correspondant, en partie supérieure et en partie inférieure de ladite conduite 3,3',3" de refroidissement et/ou de chauffage.

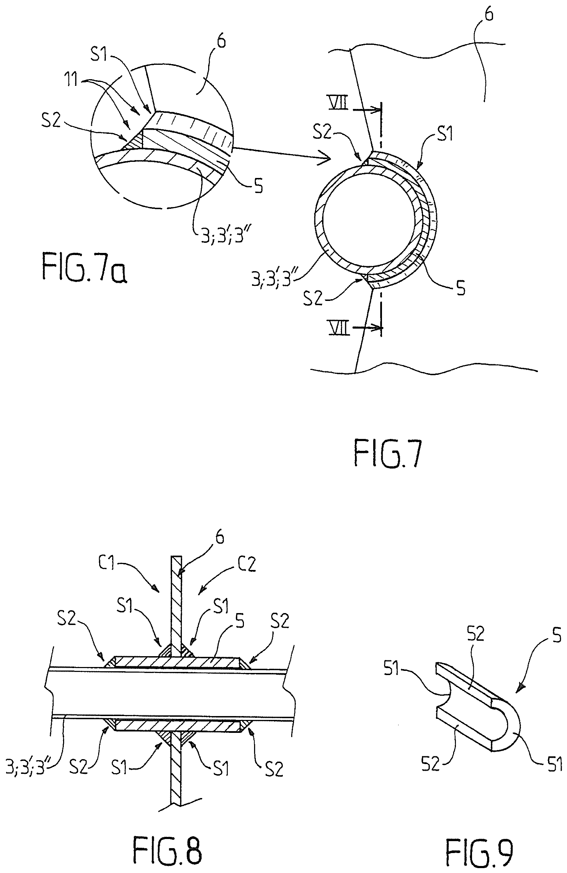

Les raccords 5 peuvent être alors de forme semi-tubulaire tels qu'illustrés à la figure 8. Dans ce cas, ladite seconde soudure S2 peut être constituée par une ligne de soudure continue, de trajectoire fermée, qui court, au niveau des bords du raccord, le long des bords longitudinaux 52 du raccord 5, ainsi que le long des bords arqués 51 dudit raccord (voir figures 5 à 8).

Alternativement les raccords 5 peuvent être de forme tubulaire tels qu'illustrés à la figure 10. Chaque raccord 5 est alors soudé à la conduite grâce à deux cordons circulaires distincts de la deuxième soudure S2 joignant les deux bords circulaires du raccord à la conduite de refroidissement et/ou de chauffage (voir figure 12).

Selon un mode de réalisation, le bioréacteur peut présenter des moyens d'agitation comprenant, en particulier :
- un rotor 8, intérieur au corps 2 de cuve, mobile en rotation par rapport au corps 2 de cuve, le rotor présentant des pales 80,
- des contre-pales 81, saillantes vers le centre de la cuve, solidarisées en des positions fixes par rapport au corps 2 de cuve.

Selon un mode de réalisation particulièrement avantageux, les contre-pales 81 desdits moyens d'agitation comprennent ledit support 6 ou lesdits supports 6 (lames 62, profilés 63..) desdits moyens de fixation. Selon ce mode de réalisation, le ou lesdits supports 6 cumulent ainsi une fonction de support de la conduite 3,3',3" de refroidissement et/ou de chauffage et une fonction de contre-pale pour l'agitation de la biomasse.
Selon un mode de réalisation avantageux, les contre-pales sont constituées entièrement par lesdits supports 6 desdits moyens de fixation, et par exemple des lames 6 selon le mode de réalisation des figures 1 à 8.

A la connaissance de la demanderesse, et dans les bioréacteurs de l'état de la technique le support de la conduite hélicoïdale de l'échangeur interne, d'une part, et les contres pales desdits moyens d'agitation, d'autre part, sont constitués par des éléments distincts. Dans ce cas, et selon l'état de l'art connu de la demanderesse, la conduite hélicoïdale est décalée radialement, bien souvent vers le centre de la conduite, par rapport aux contre-pales.

Selon ce mode de réalisation avantageux de l'invention, la conduite hélicoïdale et les supports (lames, profilés...) sont situées radialement à un même niveau, ledit support (lame, profilé...) ou lesdits supports (lames, profilés...) des moyens de fixation ne créant pas de surface supplémentaire dans la cuve, favorable aux dépôts.

De préférence, les moyens de fixation comprennent plusieurs dits supports 6 (lames 62, profilés 63...), répartis angulairement sur la circonférence interne du corps de cuve, orientés chacun suivant leur axe longitudinal selon la hauteur du corps de cuve, et s'étendant chacune au moins en correspondance sur la hauteur de l'hélicoïde.

Dans un mode préférentiel, les moyens de fixation seront répartis de manière régulière sur la circonférence interne du corps de cuve.

Le nombre et la répartition des points de réception de l'hélicoïde sont de préférence choisis comme un compromis entre contrainte à la liaison conduite/supports 6 de réception et déformation de la conduite 3,3',3" de refroidissement et/ou de chauffage. Il est souhaitable de privilégier la solution à contrainte la plus faible, tout en s'assurant que la dilation/rétractation de la conduite ne soit pas entravée lors des phases transitoires.

Les points de réception de l'hélicoïde sont de préférence régulièrement répartis le long de la conduite tous les X tours de l'hélicoïde, X pouvant être compris entre ¼ et ¾, tels que par exemple 1/3 de tours ou 2/3 de tours. En fonction de cette valeur, la conduite peut, dans certain cas, n'être pas systématiquement supportée à chaque fois que la conduite passe au voisinage des supports 6 (lame, profilé..).

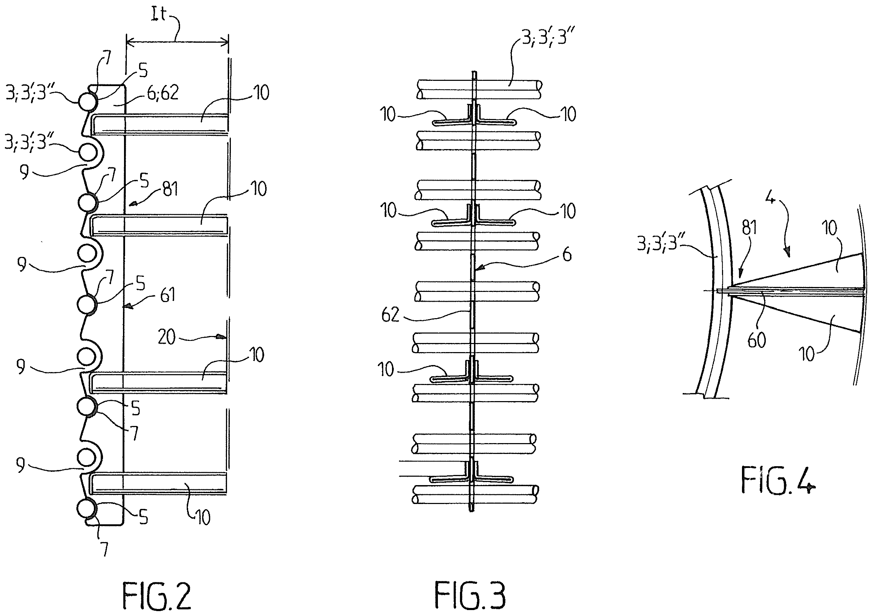

Dans ce cas, ledit ou lesdits supports 6 (lames, profilés...) peuvent présenter, outre les alésages 7, dits premiers alésages 7 au niveau desdites différentes positions de réception de l'hélicoïde, des seconds alésages 9, de dimensions supérieures par rapport au diamètre de ladite conduite. Les seconds alésages 9 sont traversés par ladite conduite de telle façon à laisser un jeu autorisant la libre déformation de ladite conduite, sans risque que la conduite ne vienne se détériorer au contact du support 6, et notamment du chant 60 de lame 62. Un tel jeu peut être supérieur à 1 cm, tel que par exemple 1,5 cm. Selon un mode de réalisation, illustré selon un exemple non limitatif à la figure 2, chaque support 6 peut présenter sur sa hauteur, en alternance, les premiers alésages 7 et les seconds alésages 9.

Dans le cas où l'une ou certaines des contre-pales 81 seraient distinctes desdits supports (lames 62, profilé 63...), la ou chaque contre-pale peut présenter de tels seconds alésages 9 afin d'être située à même rayon que le ou lesdits supports dans le corps de cuve.

De préférence, le ou les supports 6 (lames 62, profilés 63...) et/ou les contre-pales 81 desdits moyens d'agitation ne s'étendent pas directement à partir de la paroi latérale du corps de cuve, mais sont au contraire déportées de celle-ci.

Ainsi et selon un mode de réalisation illustré à la figure 2 chacun des supports 6 (lame 62, profilé 63...) présente ainsi un bord extérieur 61 en vis-à-vis de la paroi interne 20, latérale du corps de cuve et lesdits moyens de fixation 4 comprennent des contreventements 10 assurant la solidarisation du support 6 ou de chacun desdits support 6, à la paroi interne du corps de cuve, en créant un inter-espace It entre le bord extérieur 61 du support 6 correspondant et la paroi interne 20 du corps de cuve.

Un tel décalage des supports 6 et/ou des contre-pales 81 permet d'éviter la création de zones où la biomasse est stagnante dans le réacteur, lors de l'agitation, et ainsi d'assurer une agitation homogène de la biomasse au sein de la cuve.

Chaque contreventement 10 est de préférence soudé, respectivement au support 6 correspondant et à la paroi interne 20 du corps 2. Lesdites soudures, par apport de métal, présentent un cordon de métal formant un congé en creux, de rayon de préférence supérieur à 5 mm, lesdites soudures étant de préférence polies.

Le bioréacteur peut comprendre, outre ladite conduite de refroidissement/et ou de chauffage, interne au corps de cuve, un dispositif de refroidissement/chauffage du corps de cuve par l'extérieur, par circulation d'un fluide frigoporteur/caloporteur.

Ce dispositif, externe peut comprendre une double enveloppe, au corps de cuve, ou tout autre échangeur externe connu de l'homme de l'art.

Le bioréacteur conforme à l'invention trouve une application particulière pour la mise en oeuvre de procédé de fermentation de type *batch, fed-batch* ou continu et plus particulièrement la mise en oeuvre de réactions biologiques ou biochimiques sensibles aux contaminations.

Un tel réacteur est notamment destiné à la production de biomasses de cellules, et plus particulièrement la culture de microalgues, du genre *Chlorella.*

Un cycle de fermentation peut commencer par un nettoyage du bioréacteur par voie chimique et/ou physique (stérilisation), puis le remplissage du corps de cuve en substances nutritives pouvant être également stérilisées avant ou après leur introduction, puis le catalyseur biologique en particulier une biomasse cellulaire, qui est ensuite alimentée en substances nutritives, notamment carbonées, pendant la fermentation, et agitée, de préférence continuellement.

De telles réactions biologiques peuvent durer plusieurs jours. A la fin du processus, la biomasse et/ou le composé d'intérêt est récupéré par vidange de la cuve.

Le bioréacteur conforme à l'invention est avantageusement dépourvu, autant que possible, de toute zone, en particulier interstitielle au sein du corps de cuve, favorable au développement des contaminations. Le réacteur est ainsi conçu pour éviter le dépôt de substances, notamment organiques, au cours de la réaction de fermentation, par l'action notamment de l'agitation interne. Il permet avantageusement d'éviter, voire de limiter fortement les interruptions de production pour la maintenance et le nettoyage de la cuve.

Le bioréacteur peut comprendre, de manière connue en soit, une ouverture d'alimentation pour les produits et les auxiliaires (nutriments, acide/base pour régulation du pH, ajout d'antimousse...), une sortie pour l'évacuation des produits, divers capteurs, tels que capteur de pH, de températures, de gaz (O₂) ... ainsi qu'un système de contrôle/commande, voire un diffuseur d'air (connu sous le terme « spargeur ») en partie basse, des hublots, avec ou sans éclairage ou tout autre accessoire usuellement rencontré dans de tels réacteurs.

L'invention trouve une application pour la production de biomasses de cellules choisies dans le groupe constituée des cellules dite de type « sauvage » ou mutée par des techniques de type mutagenèse aléatoire ou de génie génétique.
L'invention trouve une application dans le domaine des industries alimentaires Humaines ou Animales, industries Biotechnologiques, industries Pharmaceutique et Cosmétologique, dans le domaine des Biocarburants et de la Chimie.

Naturellement, d'autres modes de réalisation auraient pu être envisagés, sans sortir du cadre de l'invention tel que défini par les revendications ci-après.

### Nomenclature

1. Bioréacteur,
2. Corps de cuve,
3, 3',3". Conduite de refroidissement/et ou de chauffage,
4. Moyens de fixation,
5. Raccords,
6. Support(s),
7. Alésages (Premiers alésages),
8. Rotor,
9. Alésages (Seconds alésages),
10. Contreventements,
11. Congé (Soudures),
20. Paroi interne,
51. Bords arqués,
52. Bords longitudinaux,
60. Chant (support 6),
61. Bord extérieur (support 6),
62. Lames (supports 6),
63. Profilé (support 6),
80. Pales (Rotor 8),
81. Contre-pales,
C1,C2. Cotés supports,
It. Interspace,
S1. Première soudure,
S2. Deuxième soudure,

## Revendications

1. Bioréacteur (1) comprenant :
- un corps (2) de cuve, dont les parois internes définissent entre elles un volume intérieur pour la réception de la biomasse,
- une conduite (3,3',3") de refroidissement et/ou de chauffage dont la paroi extérieure est destinée à être en contact direct avec la biomasse, disposée dans ledit volume intérieur, et s'étendant au moins sur une partie de longueur sous la forme d'une hélicoïde,
- des moyens de fixation (4) assurant le support de ladite une conduite (3,3',3") de refroidissement et/ou de chauffage et sa fixation au corps (2) de cuve, en plusieurs positions de réception le long de l'hélicoïde,
**caractérisé en ce que** lesdits moyens de fixation comprennent :
- des raccords (5), tubulaires ou semi tubulaires, enveloppant ladite conduite (3,3',3") par l'extérieur, localement, au niveau desdites différentes positions de réception le long de l'hélicoïde,
- un ou plusieurs supports (6), solidarisés à la paroi interne du corps (2) de cuve, saillants vers le centre du corps de cuve, ledit ou lesdits supports (6) présentant plusieurs alésages (7) de forme complémentaire aux dimensions externes des raccords (5), prévus au niveau desdits différentes positions de réception le long de l'hélicoïde, lesdits alésages (7) recevant lesdits raccords (5),
et dans lequel ladite conduite (3,3',3") de refroidissement et/ou de chauffage est solidarisée au niveau de chacune desdites positions de réception par l'intermédiaire :
- d'une première soudure (S1) reliant le raccord (5) correspondant et le support (6) correspondant, ladite première soudure (S1) s'étendant le long des bords de l'alésage (7) correspondant, des deux côtés dudit support (6) correspondant, et,
- d'une seconde soudure (S2) reliant le raccord (5) correspondant et la conduite (3, 3',3") de refroidissement et/ou de chauffage, ladite seconde soudure (S2) s'étendant le long des bords dudit raccord (5),
et dans lequel ladite première soudure (S1) et ladite deuxième soudure (S2) sont des soudures par apport de métal.

2. Bioréacteur selon la revendication 1 dans lequel le cordon de soudure ou chacun des cordons de métal de ladite première soudure (S1) et/ou de ladite deuxième soudure (S2) forme chacun un congé (11), en creux, lesdites soudures étant polies.

3. Bioréacteur selon la revendication 1 ou 2, dans lequel chacun des alésages (7) est débouchant au niveau d'un bord du support (6) ou de l'un desdits supports (6).

4. Bioréacteur selon la revendication 3, dans lequel les alésages (7) sont débouchant vers le centre du corps (2) de cuve.

5. Bioréacteur selon la revendication 3 ou 4 dans lequel ladite première soudure (S1) est constituée par une ligne de soudure continue, de trajectoire fermée, qui court, au niveau des bords de l'alésage (7), des deux côtés (C1,C2) dudit support (6) correspondant, ainsi que sur le chant (60) dudit support (6) correspondant, en partie supérieure et en partie inférieure de ladite conduite (3,3',3") de refroidissement et/ou de chauffage.

6. Bioréacteur selon l'une des revendications 3 à 5, dans lequel les raccords (5) sont de forme semi-tubulaire et dans lequel ladite seconde soudure (S2) est constituée par une ligne de soudure continue, de trajectoire fermée, qui court, au niveau des bords du raccord, le long des bords longitudinaux (52) du raccord (5), ainsi que le long des bords arqués (51) dudit raccord.

7. Bioréacteur selon l'une des revendications 1 à 6, présentant des moyens d'agitation comprenant :
- un rotor (8), intérieur au corps (2) de cuve, mobile en rotation par rapport au corps (2) de cuve, le rotor présentant des pales (80),
- des contre-pales (81), saillantes vers le centre de la cuve, solidarisées en des positions fixes par rapport au corps (2) de cuve.

8. Bioréacteur selon la revendication 7, dans lequel les contre-pales (81) desdits moyens d'agitation comprennent ledit support (6) ou lesdits supports (6) desdits moyens de fixation.

9. Bioréacteur selon l'une des revendications 1 à 8, dans lequel les alésages (7) sont dits premiers alésages, ledit ou lesdits supports (6) présentant, outre lesdits premiers alésages (7) au niveau desdites différentes positions de réception de l'hélicoïde, des seconds alésages (9), de dimensions supérieures par rapport au diamètre de ladite conduite, lesdits seconds alésages (9) étant traversés par ladite conduite de telle façon à laisser un jeu autorisant la libre déformation de ladite conduite.

10. Bioréacteur selon l'une des revendications 1 à 9, présentant plusieurs dits supports (6), répartis angulairement sur la circonférence interne du corps de cuve, orientés chacun suivant leur axe longitudinal selon la hauteur du corps de cuve, s'étendant chacune en correspondance sur la hauteur de l'hélicoïde.

11. Bioréacteur selon l'une des revendications 1 à 10 dans lequel ledit support (6) ou chacun des supports (6) présente un bord extérieur en vis-à-vis de la paroi interne (20), latérale, de la cuve (2) et dans lequel lesdits moyens de fixation (4) comprennent des contreventements (10) assurant la solidarisation dudit support (6) ou de chacun desdits supports, à la paroi interne du corps de cuve, en créant un inter-espace (It) entre le bord extérieur (61) du support (6) correspondant et la paroi interne (20) du corps de cuve.

12. Bioréacteur selon la revendication 11, dans lequel chaque contreventement (10) est soudé, respectivement au support (6) correspondant et à la paroi interne (20) du corps (2) de cuve et dans lequel lesdites soudures, par apport de métal, présentent un cordon de métal formant un congé en creux, lesdits soudures étant polies.

13. Bioréacteur selon l'une des revendications 1 à 12, pourvu d'un dispositif de refroidissement/chauffage du corps de cuve par l'extérieur, par circulation d'un fluide frigoporteur/caloporteur.

14. Bioréacteur selon l'une des revendications 1 à 13, dans lequel le ou chacun des supports (6) est choisi parmi une lame (62) ou un profilé (63).

15. Utilisation du bioréacteur selon l'une des revendications 1 à 14 pour la réalisation de réactions biologiques ou biochimiques, plus particulièrement la production de biomasses de cellules, et plus particulièrement encore la culture de microalgues.

16. Utilisation selon la revendication 15, pour la production de biomasses de cellules choisies dans le groupe constituée des cellules dite de type « sauvage » ou mutée par des techniques de type mutagenèse aléatoire ou de génie génétique.

17. Utilisation selon l'une ou l'autre des revendications 15 et 16 pour la production de microalgues du genre *Chlorella.*

18. Utilisation selon l'une quelconque des revendications 15 à 17 dans le domaine des industries alimentaires Humaines ou Animales, industries Biotechnologiques, industries Pharmaceutique et Cosmétologique, dans le domaine des Biocarburants et de la Chimie.

## Patentansprüche

1. Bioreaktor (1), umfassend:
- einen Behälterkörper (2), dessen Innenwände zwischen sich ein Innenvolumen für die Aufnahme der Biomasse definierten,
- eine Kühl- und/oder Heizleitung (3, 3', 3"), deren Außenwand dazu bestimmt ist, in direktem Kontakt mit der Biomasse zu sein, die in dem Innenvolumen angeordnet ist und sich zumindest auf einem Teil der Länge schraubenförmig erstreckt,
- Befestigungsmittel (4), die die Unterstützung der einen Kühl- und/oder Heizleitung (3, 3', 3") und ihre Befestigung am Behälterkörper (2) in mehreren Aufnahmepositionen entlang der Schraube gewährleisten,
**dadurch gekennzeichnet, dass** die Befestigungsmittel umfassen:
- röhrenförmige oder halb röhrenförmige Anschlüsse (5), die die Leitung (3, 3', 3") von außen lokal im Bereich der verschiedenen Aufnahmepositionen entlang der Schraube umhüllen,
- einen oder mehrere Träger (6), die mit der Innenwand des Behälterkörpers (2) verbunden sind, die zur Mitte des Behälterkörpers herausragen, wobei der oder die Träger (6) mehrere Bohrungen (7) von komplementärer Form zu den Außenabmessungen der Anschlüsse (5) aufweisen, die im Bereich der verschiedenen Aufnahmepositionen entlang der Schraube vorgesehen sind, wobei die Bohrungen (7) die Anschlüsse (5) aufnehmen,
und bei dem die Kühl- und/oder Heizleitung (3, 3', 3") im Bereich jeder der Aufnahmepositionen angebunden ist mit Hilfe:
- einer ersten Schweißung (S1), die den entsprechenden Anschluss (5) und den entsprechenden Träger (6) verbindet, wobei sich die erste Schweißung (S1) entlang der Ränder der entsprechenden Bohrung (7) auf beiden Seiten des entsprechenden Trägers (6) erstreckt, und
- einer zweiten Schweißung (S2), die den entsprechenden Anschluss (5) und die Kühl- und/oder Heizleitung (3, 3', 3") verbindet, wobei sich die zweite Schweißung (S2) entlang der Ränder des Anschlusses (5) erstreckt,
und bei dem die erste Schweißung (S1) und die zweite Schweißung (S2) Schweißungen mit Metallauftrag sind.

2. Bioreaktor nach Anspruch 1, bei dem die Schweißnaht oder jede der Metallnähte der ersten Schweißung (S1) und/oder der zweiten Schweißung (S2) jeweils eine Hohlkehle (11) bilden, wobei die Schweißungen poliert werden.

3. Bioreaktor nach Anspruch 1 oder 2, bei dem jede der Bohrungen (7) im Bereich eines Randes des Trägers (6) oder eines der Träger (6) mündet.

4. Bioreaktor nach Anspruch 3, bei dem die Bohrungen (7) zur Mitte des Behälterkörpers (2) hin münden.

5. Bioreaktor nach Anspruch 3 oder 4, bei dem die erste Schweißung (S1) von einer kontinuierlichen Schweißlinie mit geschlossenem Verlauf gebildet ist, die im Bereich der Ränder der Bohrung (7) von den zwei Seiten (C1, C2) des entsprechenden Trägers (6) sowie auf der Kante (60) des entsprechenden Trägers (6) im oberen Teil und im unteren Teil der Kühl- und/oder Heizleitung (3, 3', 3") verläuft.

6. Bioreaktor nach einem der Ansprüche 3 bis 5, bei dem die Anschlüsse (5) von halb-röhrenförmiger Form sind, und bei dem die zweite Schweißung (S2) von einer kontinuierlichen Schweißlinie mit geschlossenem Verlauf gebildet ist, die im Bereich der Ränder des Anschlusses entlang der Längsränder (52) des Anschlusses (5) sowie entlang der gebogenen Ränder (51) des Anschlusses verläuft.

7. Bioreaktor nach einem der Ansprüche 1 bis 6, der Bewegungsmittel aufweist, umfassend:
- einen Rotor (8) innerhalb des Behälterkörpers (2), der in Drehung in Bezug zum Behälterkörper (2) beweglich ist, wobei der Rotor Rotorblätter (80) aufweist,
- Gegenrotorblätter (81), die zur Mitte des Behälters hin herausragen und an festen Positionen in Bezug zum Behälterkörper (2) verbunden sind.

8. Bioreaktor nach Anspruch 7, bei dem die Gegenrotorblätter (81) der Bewegungsmittel den Träger (6) oder die Träger (6) der Befestigungsmittel umfassen.

9. Bioreaktor nach einem der Ansprüche 1 bis 8, bei dem die Bohrungen (7) erste Bohrungen sind, wobei der oder die Träger (6) außer den ersten Bohrungen (7) im Beriech der verschiedenen Aufnahmepositionen der Schraube zweite Bohrungen (9) mit größeren Abmessungen als der Durchmesser der Leitung aufweisen, wobei die zweiten Bohrungen (9) von der Leitung derart durchquert werden, dass ein Spiel gelassen wird, das die freie Verformung der Leitung gestattet.

10. Bioreaktor nach einem der Ansprüche 1 bis 9, der mehrere Träger (6) aufweist, die im Winkel auf dem Innenumfang des Behälterkörpers verteilt sind, die jeweils entlang ihrer Längsachse entlang der Höhe des Behälterkörpers ausgerichtet sind, und die sich jeweils entsprechend auf der Höhe der Schraube erstrecken.

11. Bioreaktor nach einem der Ansprüche 1 bis 10, bei dem der Träger (6) oder jeder der Träger (6) einen Außenrand gegenüber der seitlichen Innenwand (20) des Behälters (2) umfasst, und bei dem die Befestigungsmittel (4) Versteifungen (10) umfassen, die die Verbindung des Trägers (6) oder jedes der Träger mit der Innenwand des Behälterkörpers gewährleisten, wobei ein Zwischenraum (It) zwischen dem Außenrand (61) des entsprechenden Trägers (6) und der Innenwand (20) des Behälterkörpers erzeugt wird.

12. Bioreaktor nach Anspruch 11, bei dem jede Versteifung (10) an den entsprechenden Träger (6) bzw. die Innenwand (20) des Behälterkörpers (2) geschweißt ist, und bei dem die Schweißungen durch Metallauftrag eine Metallnaht aufweisen, die eine Hohlkehle bildet, wobei die Schweißungen poliert werden.

13. Bioreaktor nach einem der Ansprüche 1 bis 12, der mit einer Kühl-/Heizvorrichtung des Behälterkörpers von außen durch Zirkulation eines Kälte-/Wärmeträgerfluids versehen ist.

14. Bioreaktor nach einem der Ansprüche 1 bis 13, bei dem der oder jeder der Träger (6) unter einer Lamelle (62) oder einem Profil (63) ausgewählt ist.

15. Verwendung des Bioreaktors nach einem der Ansprüche 1 bis 14 für die Durchführung von biologischen oder biochemischen Reaktionen, insbesondere die Produktion von Biomassen von Zellen und im Speziellen für die Kultur von Mikroalgen.

16. Verwendung nach Anspruch 15 für die Produktion von Biomassen von Zellen, ausgewählt in der Gruppe, die von den Zellen "wilden" Typs gebildet ist, oder die durch Techniken des Typs Zufallsmutagenese oder Gentechnik mutiert ist.

17. Verwendung nach dem einen oder dem anderen der Ansprüche 15 und 16 für die Produktion von Mikroalgen der Gattung *Chlorella.*

18. Verwendung nach einem der Ansprüche 15 bis 17, auf dem Gebiet der Nahrungsmittelindustrien für Mensch oder Tier, der biotechnologischen Industrien, der pharmazeutischen und kosmetischen Industrien, auf dem Gebiet der Biokraftstoffe und der Chemie.

## Claims

1. Bioreactor (1) comprising:
- a vessel body (2), the internal walls of which define between them an internal volume for receiving the biomass,
- a cooling and/or heating conduit (3, 3', 3"), the external wall of which is intended to be in direct contact with the biomass, disposed in said internal volume, and extending at least over part of the length in the form of a helicoid,
- fixing means (4) supporting said cooling and/or heating conduit (3, 3', 3") and fixing it to the vessel body (2), in a plurality of reception positions along the helicoid, **characterised in that** said fixing means comprise:
- couplings (5), tubular or semi-tubular, enveloping said conduit (3, 3', 3") from the outside, locally, at said various reception positions along the helicoid,
- one or more supports (6), secured to the internal wall of the vessel body (2), projecting towards the centre of the vessel body, said support or supports (6) having a plurality of bores (7) with a form complementary to the external dimensions of the couplings (5), provided at said various reception positions along the helicoid, said bores (7) receiving said couplings (5),
and wherein said cooling and/or heating conduit (3, 3', 3") is secured at each of said reception positions by means of:
- a first weld (S1) connecting the corresponding coupling (5) and the corresponding support (6), said first weld (S1) extending along the edges of the corresponding bore (7), on both sides of said corresponding support (6), and
- a second weld (S2) connecting the corresponding coupling (5) and the cooling and/or heating conduit (3, 3', 3"), said second weld (S2) extending along the edges of said coupling (5),
and wherein said first weld (S1) and said second weld (S2) are welds by the addition of metal.

2. Bioreactor according to claim 1, wherein the weld bead or each of the metal beads of said first weld (S1) and/or of said second weld (S2) each form a hollow fillet (11), said welds being polished.

3. Bioreactor according to claim 1 or claim 2, wherein each of the bores (7) emerges at an edge of the support (6) or of one of said supports (6).

4. Bioreactor according to claim 3, wherein the bores (7) emerge towards the centre of the vessel (2).

5. Bioreactor according to claim 3 or claim 4, wherein said first weld (S1) consists of a continuous weld line, with a closed pathway, which, at the edges of the bore (7), runs along the two sides (C1, C2) of said corresponding support (6), as well as on the edge (60) of said corresponding support (6), at the top part and at the bottom part of said cooling and/or heating conduit (3, 3', 3").

6. Bioreactor according to any of claims 3 to 5, wherein the couplings (5) are semi-tubular in form and wherein the second weld (S2) consists of a continuous weld line, with a closed pathway, which, at the edges of the coupling, runs along the longitudinal edges (52) of the coupling (5), as well as along the arched edges (51) of said coupling.

7. Bioreactor according to any of claims 1 to 6, having stirring means comprising:
- a rotor (8), internal to the vessel body (2), able to rotate with respect to the vessel body (2), the rotor having blades (80),
- counter-blades (81), projecting towards the centre of the vessel, secured at fixed positions with respect to the vessel body (2).

8. Bioreactor according to claim 7, wherein the counter-blades (81) of said stirring means comprise said support (6) or said supports (6) of said fixing means.

9. Bioreactor according to any of claims 1 to 8, wherein the bores (7) are said to be first bores, said support or supports (6) having, apart from said first bores (7) at said various reception positions of the helicoid, second bores (9), with larger dimensions with respect to the diameter of said conduit, said second bores (9) having said conduit pass through them so as to leave clearance allowing free deformation of said conduit.

10. Bioreactor according to any of claims 1 to 9, having a plurality of said supports (6), distributed angularly over the internal circumference of the vessel body, each oriented along their longitudinal axis over the height of the vessel body, each extending correspondingly over the height of the helicoid.

11. Bioreactor according to any of claims 1 to 10, wherein said support (6) or each of the supports (6) has an external edge facing the lateral internal wall (20) of the vessel (2), and wherein said fixing means (4) comprise bracings (10) providing the securing of said support (6) or of each of said supports, to the internal wall of the vessel body, creating an interspace (It) between the external edge (61) of the corresponding support (6) and the internal wall (20) of the vessel body.

12. Bioreactor according to claim 11, wherein each bracing (10) is welded, respectively to the corresponding support (6) and to the internal wall (20) of the vessel body (2), and wherein said welds, by the addition of metal, have a bead of metal forming a hollow fillet, said welds being polished.

13. Bioreactor according to any of claims 1 to 12, provided with a device for cooling/heating the vessel body from outside, by the circulation of a cold-transfer / heat-transfer fluid.

14. Bioreactor according to any of claims 1 to 13, wherein the or each of the supports (6) is chosen from a plate (62) or a profiled section (63).

15. Use of the bioreactor according to any of claims 1 to 14, for carrying out biological or biochemical reactions, more particularly the production of cell biomasses, more particularly still the culture of microalgae.

16. Use according to claim 15, for producing biomasses of cells chosen from the group consisting of cells said to be of the "wild" type or mutated by techniques of the random mutagenesis or genetic engineering type.

17. Use according to either of claims 15 and 16 for producing microalgae of the *Chlorella* genus.

18. Use according to any of claims 15 to 17 in the field of human or animal food industries, biotechnology industries, pharmaceutical and cosmetological industries and in the biofuel and chemistry field.
